Europäisches Patentamt

European Patent Office

Office européen des brevets

(19)

(11) Publication number: **0 139 127**
**A1**

(12) # EUROPEAN PATENT APPLICATION

(21) Application number: **84109290.1**

(22) Date of filing: **06.08.84**

(51) Int. Cl.⁴: **A 61 K 9/26**
**A 61 K 9/70**

(30) Priority: **22.08.83 US 525089**

(43) Date of publication of application:
**02.05.85 Bulletin 85/18**

(84) Designated Contracting States:
**AT BE CH DE FR GB IT LI NL**

(71) Applicant: **F. HOFFMANN-LA ROCHE & CO.**
**Aktiengesellschaft**

**CH-4002 Basel(CH)**

(72) Inventor: **Touitou, Elka**
**54 Kubovy**
**Jerusalem(IL)**

(72) Inventor: **Goldberg, Arthur H.**
**143 Montclair Avenue**
**Montclair, N.J.(US)**

(74) Representative: **Grossner, Lutz, Dr. et al,**
**Grenzacher Strasse 124 Postfach 3255**
**CH-4002 Basel(CH)**

(54) **Transdermal drug delivery device and its preparation.**

(57) There is disclosed a solvated compressed hydrophilic matrix for transdermal delivery of a drug to warm-blooded animals. In addition to the drug, the matrix comprises 20% to 100% by weight of a directly compressible hydrophilic polymer which is solvated with 20% to 70% by weight of a hydrophilic solvent. The compressed matrix may be in the form of a compressed tablet or a two-layered compressed tablet.

EP 0 139 127 A1

Croydon Printing Company Ltd.

Transdermal drug delivery device and its preparation

Different theoretical and technical approaches have been employed to develop pharmaceutical dosage forms for transdermal controlled administration of potent drugs for systemic therapeutic effects. Several transdermal delivery systems which have been developed include the use of rate-controlling membranes (U.S. Patent Nos. 3,996,939 and 4,031,894); a polymeric diffusion matrix (European Patent Application No. 13,606); and a gel diffusion matrix (U.S. Patent No. 4,336,243). The foregoing are approaches to controlled drug delivery that yield acceptably predictable drug availability for relatively long duration, i.e. 24 hours and in some instances greater than 48 hours.

Synthetic high molecular weight hydrophilic polymers of acrylic acid, referred to as carbomers, have been used in pharmaceutical applications as a thickening and suspending agent, a bulk laxative, a binder for tablets and as a reactive agent for preparing medicinal salts.

It has now been discovered that a pharmaceutical transdermal delivery device can be prepared by solvating a compressed hydrophilic polymer containing a drug.

The invention relates to solvated or hydrated compressed matrices for transdermal administration of a drug for use in human and veterinary medicine. These compositions are suitable for establishing therapeutically active blood or skin levels of drug. The matrix comprises a hydrophilic polymer capable of being solvated with a hydrophilic solvent to produce a flexible matrix containing a predetermined amount of liquid, drug and, optionally, compatible

Grn/24.7.84

additives.

More particularly, the invention relates to a transdermal drug delivery device comprising a therapeutically effective amount of a drug and from 20 to essentially 100% by weight of a hydrophilic polymer said hydrophilic polymer being a synthetic water soluble polymer of acrylic acid cross-linked with allyl sucrose containing 56-68% carboxylic groups and having a molecular weight range of from about 1,250,000 to about 4,000,000 which polymer is solvated with 20 to 70% by weight of a hydrophilic solvent.

The polymeric materials utilized in preparing the transdermal device of the present invention are characterized as being hydrophilic and possessing suitable flow properties to allow direct compression of the polymer to a tablet or disk configuration. They are water soluble materials which are directly compressible to a tablet or disk configuration. These resins are known as carbomer resins and are available in the trade under the designation Carbopol$^R$ resins (a trademark of B.F. Goodrich). The preferred members of this class of resins are Carbopol$^R$ 934 P, 940 and 941. The molecular weight of these materials range about 1,250,000 to about 4,000,000.

The polymeric matrix may contain other hydrophilic polymers in addition to the carbomer resin. Suitable hydrophilic polymers which may be used in combination with carbomer resins include algin, xanthan gum, polyvinyl alcohol, polyvinyl pyrrolidone and hydrophilic cellulose ethers, for example methyl cellulose, hydroxypropyl cellulose, polypeptides for example, gelatins.

When one of the above-noted hydrophilic polymers is used in combination with the carbomer resin, it is present from about 1 to about 50% by weight of the total dry weight of the matrix.

The dry compressed matrix is solvated with various solvents and solvent systems. Examples of solvents which are useful in preparing the device of this invention are water, buffered aqueous solutions, triethanolamine solution, organic amines, for example, ethomine, and aqueous or hydroalcoholic solutions.

It has also been found advantageous to use aqueous solvent systems containing hydrophilic organic solvents or surfactants to solvate the compressed matrix.

Hydrophilic organic solvents suitable for solvating the compressed matrix include glycerin, propylene glycol polyethylene glycol and the like. Mixtures of from about 0.5% to about 50% by weight of a hydrophilic organic solvent are useful. A mixture of glycerin and water, e.g. 20% by weight glycerin in water, allows good retention of the solvent in the matrix, and improves the flexibility and the adherence of the device to the skin.

Aqueous solutions of surfactants have also been found useful to solvate the compressed matrix. Suitable surfactants which are useful in the practice of this invention include anionic surfactants, for example, sodium alkylsulfonates; nonionic surfactants, for example, polysorbate 80 (polyoxyethylene 20 sorbitan monooleate); and cationic surfactants, for example, cetyl pyridinium chloride and benzalkonium chlorides. Aqueous solutions comprising about 0.5 to about 25% by weight of surfactant are useful to solvate the compressed matrix compositions.

The amount of drug incorporated in the delivery device of the present invention to obtain the desired therapeutic effect will vary depending upon the desired dosage and the length of time the device is to remain in contact with the skin. The drug may be present in dosages from about 0.01 mg to 1.0 gram for systemic or topical administration. Generally, the loading dose exceeds the available dose. A ten-fold excess of drug may be optimal, however, the dose may vary from 1 to 20 times the dose released.

In practicing this invention one can employ any drug which will be absorbed by the skin to which the device is applied, or any drug that benefits the patient, even locally. The amount of drug necessary to obtain the desired therapeutic effect will vary depending on the particular drug used.

Suitable drugs which can be administered by the devices of the invention include:

diuretics, for example, benzthiazide, chlorothiazide, hydrochlorothiazide, cyclothiazide, flumethiazide, furo-semide, triamterene, ethacrynic acid, bumetanide; and the like;

sedatives and hypnotics, for example, barbiturates, such as phenobarbital, sodium phenobarbital, secobarbital, sodium pentabarbital, and the like; chloral hydrate; glutethimide, methylprylon, methaqualone and the like;

tranquilizers, such as chloropromazine, promazine, pro-chlorperazine, reserpine, meprobamate, benzodiazepines, such as chlordiazepoxide, diazepam, oxazepam, bromazepam, and the like;

antibiotics such as penicillins, cephalosporins, tetra-cyclines, oxytetracyclines, chlorotetracycline, chloram-phenicol, streptomycin, neomycin and the like;

antibacterials, such as sulfonamides, phenolics, mer- curials, quarternary ammonium compounds, and the like;

analgesics, such as codeine, morphine, meperidine, phenazocine, propoxyphene, pentazocine, and the like;

antipyretics and anti-inflammatory agents, such as aspirin, salicylamide, naproxen, indomethacin, fenoprofen, indoprofen, sulidac, diclofenac, carprofen, and the like;

antitumor agents, such as 5-fluorouracil, floxuridine, cyclophosphamide, estramustine phosphate cytosine arabinoside, and the like;

cardiovascular drugs, such as nitroglycerin, amyl nitrite, pentaerythritol tetranitrate, isosorbide dinitrate, dipyridamole, propranolol, digitalis, digitoxin, digoxin, and the like;

anti-arrhythmic drugs, such as quinidine, lidocaine, procainamide, disopyramide and the like;

hypotensive drugs, such as hydralazine and the like;

anticonvulsants, such as phenytoin, methsuximide, clonazepam, carbamazepine, ethotoin, clonopin, and the like;

antiulcer drugs, such as cimetidine; ranitidine, and the like;

hypoglycemic drugs, such as the thiazides, and the like;

vitamin compounds and derivatives thereof, such as calcitriol and the like;

anti-Parkinsonism drugs, such as levodopa, methyldopa, trihexypenidyl, cycrimine, anatadine; and the like.

It is also contemplated that more than one drug may be incorporated in the same device of the present invention. Mixtures of drugs may be compressed together with the carbomer or with other hydrophilic polymers found useful for the present invention.

The drug delivery device of this invention may also contain additives to improve the physical form or the performance of the device. Additives which may be used include diluents, such as, lactose, mannitol, dry starch; and the like; binders such as starch, natural and synthetic gums, and the like; lubricants, such as stearic acid, magnesium stearate, and the like; preservatives, such as, methyl paraben, propyl paraben, benzoic acid, and the like.

The device of the present invention may also contain a topical drug enhancer; that is, a material which assists penetration of the drug through the skin. The penetration enhancers suitable for the purpose of the invention are the therapeutically acceptable penetration enhancers that do not adversely affect the drug, the host, or alter the materials forming the drug delivery device. Examples of penetration enhancers include 1-dodecylazacycloheptan-2-one, propyleneglycol, and surfactants.

In accordance with the present invention the transdermal drug delivery device is prepared by

a) forming a mixture of a therapeutically effective amount of a drug with about 20 to about 100% by weight of said hydrophilic polymer,

b) compressing said mixture into a tablet configuration,

c) solvating said compressed tablet with a hydrophilic solvent, and

d) removing said hydrophilic solvent to provide said hydrophilic polymer with a solvation degree of from about 20 to about 70% by weight of said hydrophilic solvent.

In step a) the drug ingredient is mixed with the hydrophilic polymer using conventional mixing techniques utilized in the preparation of pharmaceutical tablets. After sufficient mixing of the ingredients, conventional direct compression or granulation tablet technology is employed in step b) produce a tablet matrix of drug and hydrophilic polymer, or drug hydrophilic polymer and additives. A flat surface die of about one inch diameter affords a convenient compressed tablet matrix. It has also been found advantageous to use a die which produces a tablet or disk with a hole in the center. The hole in the tablet or disk allows one to carry out the heating or curing process more conveniently. For example, a tablet containing a hole can be prepared by core rod tooling on a Menesty Type F3 machine. In compressing the ingredients, pressures of from about 160 to about 960 bar, preferably about 320 to 800 bar are suitable for preparing the compressed matrixes of the present invention.

In step c) the compressed tablet matrix is solvated with a hydrophilic solvent or solvent system to produce a flexible gelled matrix tablet. The solvents and/or solvent systems useful in the practice of this invention are described hereinabove. The dry compressed matrix is solvated under controlled conditions. Solvation is carried out by immersing the dry compressed matrix tablet in the solvent or solvent system at room temperature or higher temperatures. The time the compressed matrix is immersed in the solvent or solvent system may vary from about 1 minute to about 60 minutes.

In step d) the excess solvent is removed from the solvated compressed matrix under controlled drying conditions.

The solvent can be removed by heating the solvated matrix at an elevated temperature depending on the stability of the drug and on the gelation properties of the polymer. Removal of the solvent can be accomplished by heating with microwaves, forced air or under ambient conditions. The temperature range for removing excess solvent can range from about room temperature to about 100°C, preferably about 70°C when the solvent is water. The heating process can vary from several minutes to two hours. Generally, a one hour heating period is sufficient to afford a satisfactory product. The degree of solvation may also be controlled by using a vacuum apparatus or vacuum oven.

In accordance with another embodiment of the present invention, the device for transdermal drug delivery may be in the form of a two-layered compressed tablet. One layer comprises a hydrophilic polymer, for example, the carbomer resin disclosed hereinabove, and a second layer comprises the hydrophilic polymer, i.e. carbomer, and drug. This configuration affords a matrix which contains a high concentration of drug in a thin second layer of carbomer.

The two-layered matrix is prepared by the techniques disclosed hereinabove for the single-layered matrix. For example, the two-layered matrix may be prepared by successive compression of the carbomer resin alone followed by compression of the drug containing carbomer resin. The two layers may be compressed under similar or different compression forces. For example, compression of the first layer of carbomer may be compressed about 80 bar and the second layer may be compressed at about 320 bar.

In accordance with another embodiment of the present invention, the device for transdermal drug delivery may be in the form of a two-layered compressed tablet composition. One layer of the two-layered tablet comprises a hydrophilic polymer containing the drug, for example, the carbomer resin disclosed hereinabove; and a second layer comprises a hydrophobic polymer or mixture of hydrophobic polymers. The carbomer resin functions as a hydrophilic reservoir and the hydrophobic layer functions as a release rate limiting layer. Suitable polymers which can be used as the second hydrophobic layer include cellulose derivatives, for example cellulose ethers, such as, ethyl cellulose, and the like, or other hydrophobic materials that can be bound by incorporation of other materials. The hydrophobic layer may also contain various proportions of the hydrophilic carbomer resin. The carbomer resin imports elasticity and adhesivity to the hydrophobic layer which otherwise may disintegrate during the solvation process.

The two-layered transdermal matrix in a dry state comprises from about 5-90% by weight of hydrophilic polymer and from about 95-10% by weight of hydrophobic polymer or polymers.

The two-layered matrixes are prepared by techniques disclosed hereinabove for the single-layered matrix. For example, the doubled-layered matrix may be prepared by successive compression of the hydrophilic polymer, i.e. carbomer, and the hydrophobic polymer or mixture of polymers. The two layers may be compressed under similar or different compression forces. For example, compression of the first layer of hydrophobic resins may be compressed at about 80 bar whereas, the second layer is compressed at about 320 bar pressure.

In accordance with another embodiment of the present invention, the device for transdermal drug delivery may be covered with a rate controlling membrane or film. Examples of rate controlling membranes and films which are useful in the practice of the present invention include a polyurethane film (available in the trade under the designation

Tegaderm^R, a trademark of 3M Company).

The drug delivery device of the present invention requires a means for attaching the matrix to the desired site of the patient. It will be apparent to those skilled in the art that many and varied means may be employed for securing the device of this invention onto the patient at the desired location. Such means can take various forms, such as an occlusive backing layer forming a kind of "bandage" with the drug delivery device being held against the skin of a patient being treated. Any adhesive bandage tape is contemplated as being useful with the present invention. It can also take the form of an elastic band, such a cloth band, a rubbery band, or other material.

The Examples which follow further illustrate this invention.

## Example 1

900 mg of carbomer resin, Carbopol^R 934, and 10 mg of bumetanide, 3-(butylamino)-4-phenoxy-5-sulfamoylbenzoic acid, were thoroughly mixed together and the mixture compressed in a Carver Laboratory Press at a pressure of about 320 bar using a flat surface die of one inch diameter. The compressed tablet matrix was soaked in water for 30 minutes at room temperature. After removal from the water, the matrix was heated at 70°C for one (1) hour in an oven. After fifty (50) minutes of heating, the matrix was reversed to permit homogeneous heating. The resulting solvated matrix was a flexible gelled tablet which maintained the original shape and had a gum like appearance.

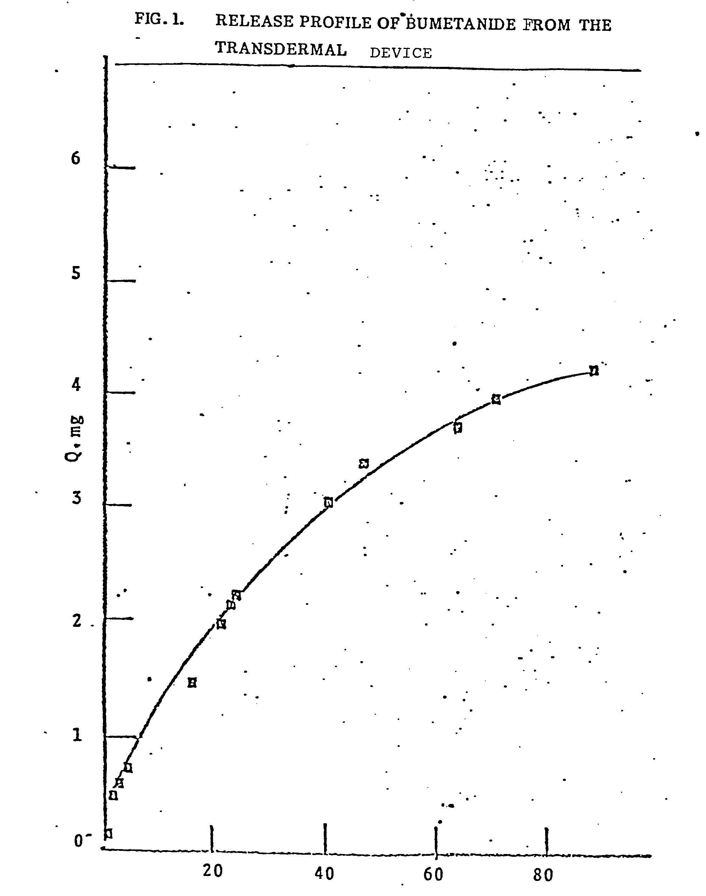

The thus formed solvated tablet was tested for in vitro release rates by the rotating disk technique and apparatus as described by Touitou et al., Intern. J. Pharm. 9 97-106 (1981). The disk of the tablet holder was modified by placing a gauze on the screen over the holder component. The release profile of bumetanide in vitro in 300 ml water at 35°C and 150 RPM is illustrated in Figure 1.

## Example 2

900 mg of carbomer resin, Carbopol$^R$ 934, and 10 mg of bumetanide, 3-(butylamino)-4-phenoxy-5-sulfamoylbenzoic acid, are thoroughly mixed together and the mixture was compressed in a Carver Laboratory Press at a pressure of about 320 bar using a flat surface die of one inch diameter. The compressed table matrix was soaked in water for 30 minutes at room temperature. After removal from the water, the matrix was heated at 70°C for one (1) hour in an oven. After fifty (50) minutes of heating, the matrix was reversed to permit homogeneous heating.

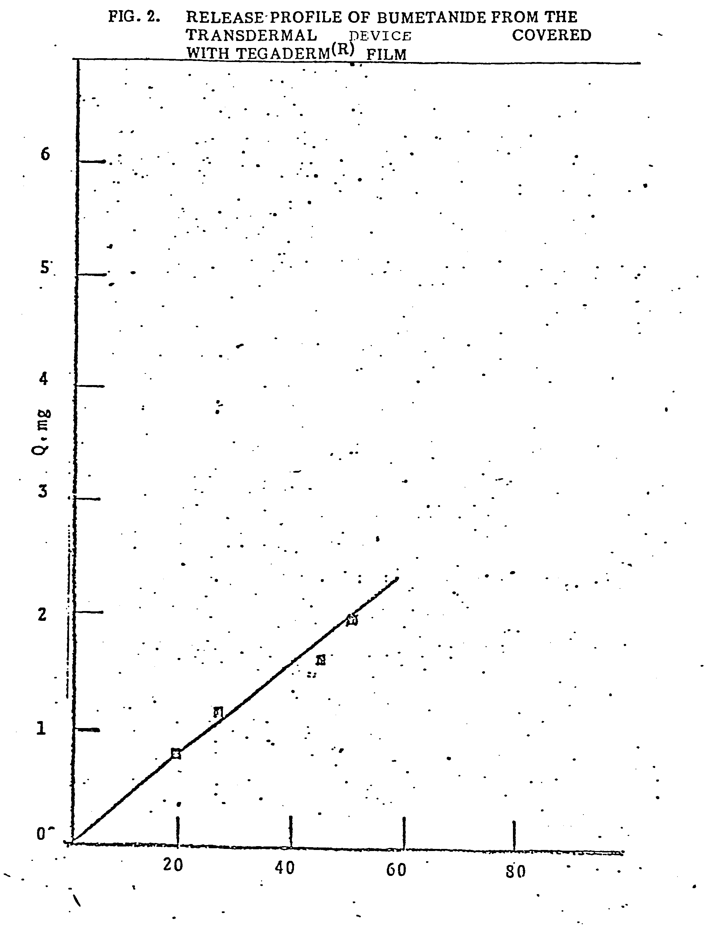

The solvated compressed matrix was completely covered with a polyurethane film (Tegaderm$^R$) and the in vitro release rate determined by the method described by Touitou et al., cited above. The release profile of the product in vitro in 300 ml water at 35°C and 150 RPM is illustrated in Figure 2.

## Example 3

900 mg of carbomer resin, Carbopol$^R$ 934, and 50 mg of diazepam, were thoroughly mixed together and the mixture was compressed in a Carver Laboratory Press at a pressure of about 350 bar using a flat surface die of one inch diameter. The compressed tablet matrix was soaked in water for thirty (30) minutes at room temperature. After removal from the water, the matrix was heated at 70°C for one (1) hour in an oven. After heating for 45 minutes, the matrix was reversed to permit homogeneous heating.

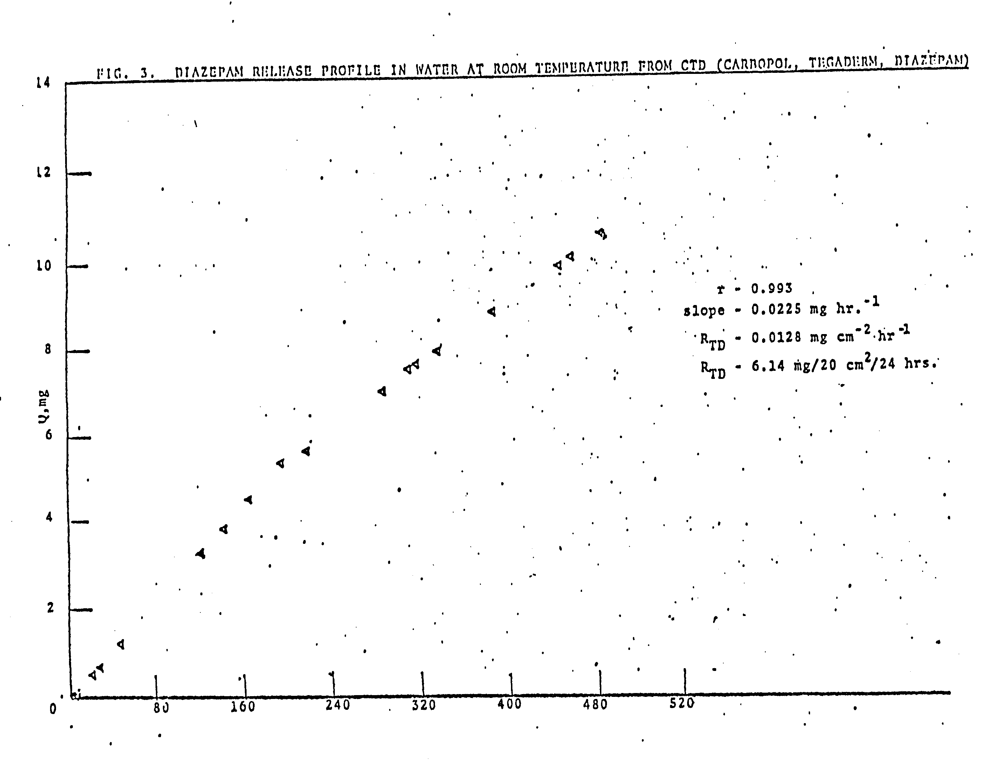

The solvated compressed matrix was completely covered with a polyurethane film (Tegaderm[R]) and the in vitro release rate determined by the rotating disk technique described by Touitou et al., cited above. The release profile of the product in vitro in 300 ml water at 35°C and 150 RPM is illustrated in Figure 3.

## Example 4

800 mg of carbomer resin Carbopol[R] 934, 150 mg of hydroxypropyl cellulose (Klucel EF) and 20 mg of bumetanide, were thoroughly mixed together. The mixture was compressed in a Carver Laboratory Press at a pressure of about 320 bar using a flat die of one inch diameter. The compressed tablet matrix was soaked in a solvent mixture of propylene glycol: water 3:5, for 40 minutes at room temperature. After removal from the solvent mixture, the solvated matrix was heated at 70°C for 25 minutes. The resulting solvated matrix was a flexible gelled tablet which maintained the original shape and had a gum like appearance.

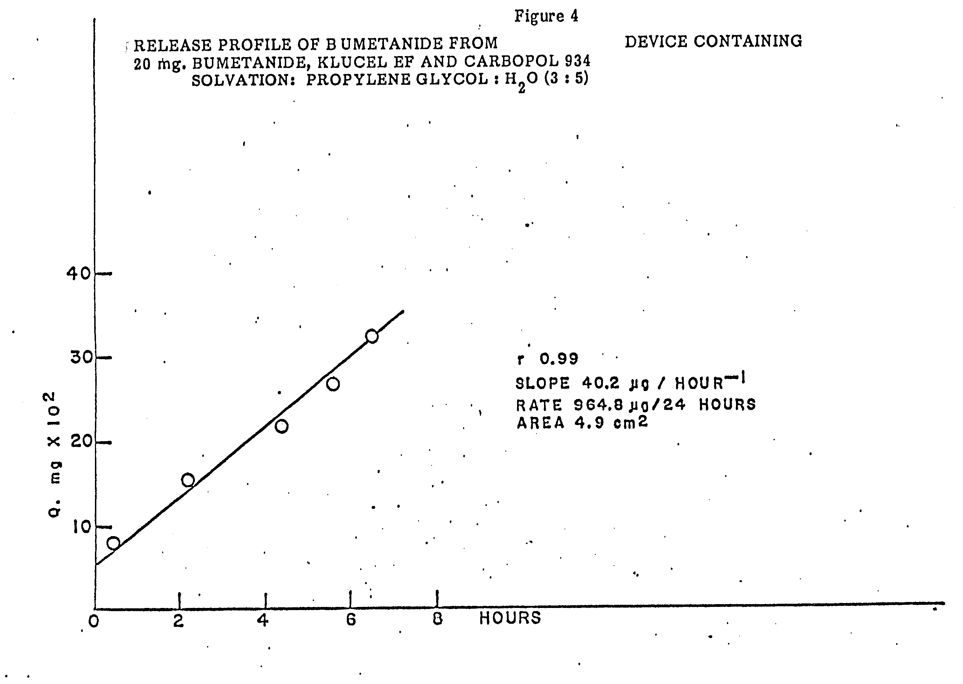

The release profile of the product in vitro is illustrated in Figure 4. The produce and apparatus described in Touitou et al., loc. citation, was utilized and assayed by fluorometry in pH 2.9 glycine buffer at 350-450 mm.

## Example 5

55 mg of bumetanide thoroughly mixed with 4500 mg of carbomer, Carbopol[R] 934. 910 mg of the mixture was compressed in a Carver Laboratory Press at a pressure of about 320 bar using a flat surface die of one inch diameter. The compressed tablet matrix was solvated by soaking it for 1 minute in water and 45 minutes at 30°C in an aqueous solution of Polysorbate 80 (25% by volume). After removal from the solution, the solvated matrix was heated at 70°C in an oven for 30 minutes. The degree of solvation for the

- 13 -

gelled matrix was calculated to be 44.3%.

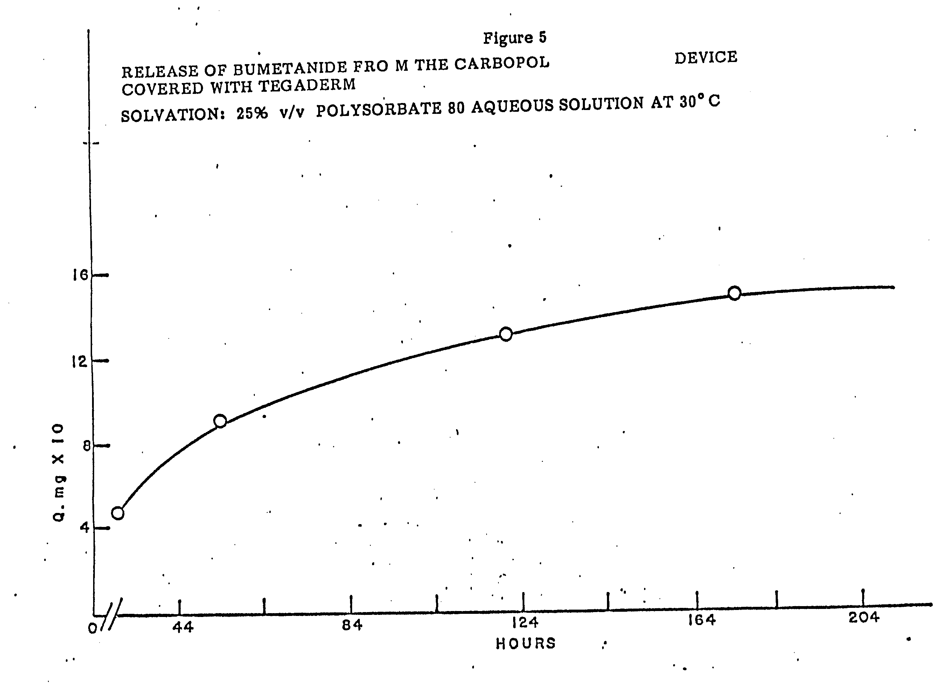

The release profile of the product in vitro is illustrated in Figure 5.

### Example 6

800 mg of carbomer resin, Carbopol[R] 934, is compressed in a Carver Laboratory Press at a pressure of 500 kg. A mixture of 10 mg of (15R,16R)-16-fluoro-15-hydroxy-9-oxo-prosta-5(Z), 13(E)-dienoic and 150 mg of carbomer resin, Carbopol[R] 934 acid is prepared and the mixture poured on the compressed carbomer layer. The materials are compressed at a pressure of about 320 bar. The two-layered compressed matrix is soaked in a mixture of propylene glycol and water at room temperature for 45 minutes. After removal from the solvent mixture, the matrix is heated at 70°C for 30 minutes.

By using the aboved noted procedure, a two-layered compressed tablet matrix is prepared. The compressed matrix is solvated by soaking it for 45 minutes in an emulsion containing 20% by weight of 1-dodecylazacycloheptan-2-one (a drug penetration enhancer), 10% by weight of Polysorbate 80 (nonionic surfactant) and water to 100%. After removel from the emulsion, the matrix is heated at 30°C for two hours.

### Example 7

100 mg of bumetanide was thoroughly mixed with 9500 mg of carbomer resin, Carbopol[R] 934. Several samples are prepared in the following manner: 950 mg of the mixture was compressed in a Carver Laboratory Press at a pressure of about 320 bar using a flat surface die of one inch diameter. The compressed tablet matrix was solvated by soaking the compressed matrix for 30 minutes at room temperature in various solvation systems. After removal from the solvation system, the matrix was heated for 30 minutes in an oven at 70°C. The degree of solvation (H) value in the various solvation systems was determined by the formula:

$$H = \frac{Wt - Wo}{Wo} \times 100$$

wherein

H = Degree of solvation
Wt = Weight of the cured matrix
Wo = Weight of the dry compressed matrix

The degree of solvation (H) values are tabulated in Table I:

### Table I

| Solvation System | H Values |
|---|---|
| Water | 86.5 |
| NaOH-0.1N | 175.5 |
| Propylene glycol: Water 2 : 5 | 59.4 |
| Propylene glycol: Water 3 : 5 | 58.5 |
| 25% aqueous solution of Polysorbate 80 | 57.6 |

Solvation with Polysorbate 80 aqueous solution was carried out at 30°C.

### Example 8

The degree of solvation (H) in water and in propylene glycol of compressed matrixes of different compositions were determined in the following manner:

The carbomer resin or mixture of carbomer resin and a hydrophilic polymer was compressed in a Carver Laboratory Press at a pressure of about 320 bar. The compressed tablet matrix was soaked in the solvent for 40 minutes at room temperature. After removal from the solvent, the matrix was heated at 70°C for 50 minutes. The degree of solvation values (H) of the various compositions is water and in propylene glycol is tabulated in Table II.

Table II

| Components | % w/w | H% | |
|---|---|---|---|
| | | Water | Propylene Glycol |
| Carbopol R 940 | 100 | 72 | 43 |
| Carbopol R 941 | 100 | 77 | 40 |
| Carbopol$^R$ 934<br>Sodium Alginate | 50<br>50 | 48 | 29 |
| Carbopol$^R$ 934<br>Xanthane Gum | 50<br>50 | 125 | 26 |
| Carbopol$^R$ 934<br>Cellulose gum<br>CMC7LF | 50<br><br>50 | 48 | 24 |
| Carbopol$^R$ 934<br>N-Glucamine | 80<br>20 | 120 | 58 |
| Carbopol$^R$ 934*<br>Bico "E" (gelatin) | 50<br>50 | 53 | 97 |

* This matrix was cured at room temperature for 50 minutes.

CLAIMS:

1. A transdermal drug delivery device comprising a therapeutically effective amount of a drug and from 20 to essentially 100% by weight of a hydrophilic polymer said hydrophilic polymer being a synthetic water soluble polymer of acrylic acid cross-linked with allyl sucrose containing 56-68% carboxylic groups and having a molecular weight range of from about 1,250,000 to about 4,000,000 which polymer is solvated with 20 to 70% by weight of a hydrophilic solvent.

2. The device of claim 1 wherein the hydrophilic polymer also comprises from about 1 to about 50% by weight of a second hydrophilic polymer selected from the group consisting of algin, xanthan gum, polyvinyl alcohol, polyvinyl pyrrolidone, methyl cellulose, hydroxypropyl cellulose and gelatin.

3. The device of claim 1 wherein the hydrophilic solvent is selected from the group consisting of water, buffered aqueous solutions, triethanolamine solution, organic amines, aqueous alcoholic solutions, aqueous solutions of glycerin, or propylene glycol, or polyethylene glycol, and aqueous solutions of anionic, or cationic or nonionic surfactants.

4. The device of claim 1 wherein the hydrophilic solvent is water.

5. The device of claim 1 wherein the hydrophilic solvent is an aqueous solution of from about 0.5% to about 50% by weight of propylene glycol.

6. The device of claim 1 wherein the hydrophilic solvent is an aqueous solution of 0.5% to 25% by weight of a nonionic surfactant.

7. The device of claim 6 wherein the nonionic surfactant is polyoxyethylene 20 sorbitan monooleate.

8. The device of claim 1 wherein said device is covered with a rate controlling membrane or film.

9. The device of claim 8 wherein said membrane or film is a polyurethane film.

10. The device of claim 1 wherein said device has a two-layered configuration, a first layer comprising a portion of the hydrophilic polymer and a second layer comprising the drug and the remainder of the hydrophilic polymer.

11. A transdermal drug delivery device comprising a two layered configuration, a first layer comprising a hydrophobic polymer and a second layer comprising a therapeutically effective amount of a drug in a hydrophilic polymer being a synthetic water soluble polymer of acrylic acid cross-linked with allyl sucrose containing 56-68% carboxylic groups and having a molecular weight range of from about 1,250,000 to about 4,000,000 said device in a dry state comprises from about 5-90% by weight of hydrophilic polymer and from about 9.5-10% by weight of hydrophilic polymer, and the total amount of hydrophobic and hydrophilic polymers in the matrix is about 20 to about 100% by weight, and the polymer matrix is solvated with about 20 to about 70% by weight of a hydrophilic solvent.

12. The matrix of claim 11 wherein the hydrophobic polymer is ethyl cellulose.

13. The device of claim 11 wherein the hydrophilic solvent is selected from the group consisting of water, buffered aqueous solutions, triethanolamine solution, organic amines, aqueous alcoholic solutions, aqueous solutions of glycerin, or propylene glycol, or polyethylene glycol, and aqueous solutions of anionic, or cationic or nonionic surfactants.

14. The device of claim 11 wherein the hydrophilic solvent is water.

15. The device of claim 11 wherein the hydrophilic solvent is an aqueous solution of from about 0.5% to about 50% by weight of propylene glycol.

16. The device of claim 11 wherein the hydrophilic solvent is an aqueous solution of 0.5% to 25% by weight of a nonionic surfactant.

17. The device of claim 16 wherein the nonionic surfactant is polyoxyethylene 20 sorbitan monooleate.

18. A process for preparing the device as claimed in claim 1 which comprises

a) forming a mixture of a therapeutically effective amount of a drug with about 20 to about 100% by weight of said hydrophilic polymer,

b) compressing said mixture into a tablet configuration,

c) solvating said compressed tablet with a hydrophilic solvent, and

d) removing said hydrophilic solvent to provide said hydrophilic polymer with a solvation degree of from about 20 to about 70% by weight of said hydrophilic solvent.

***

**FIG. 1.   RELEASE PROFILE OF BUMETANIDE FROM THE TRANSDERMAL   DEVICE**

2/5

0139127

FIG. 2.　RELEASE PROFILE OF BUMETANIDE FROM THE
　　　　　TRANSDERMAL　DEVICE　　　　COVERED
　　　　　WITH TEGADERM(R) FILM

FIG. 3. DIAZEPAM RELEASE PROFILE IN WATER AT ROOM TEMPERATURE FROM CTD (CARBOPOL, TEGADERM, DIAZEPAM)

$r = 0.993$
slope $= 0.0225$ mg hr.$^{-1}$
$R_{TD} = 0.0128$ mg cm$^{-2}$ hr$^{-1}$
$R_{TD} = 6.14$ mg/20 cm$^2$/24 hrs.

Figure 4

RELEASE PROFILE OF BUMETANIDE FROM      DEVICE CONTAINING
20 mg. BUMETANIDE, KLUCEL EF AND CARBOPOL 934
SOLVATION: PROPYLENE GLYCOL : $H_2O$ (3 : 5)

r 0.99
SLOPE 40.2 µg / HOUR$^{-1}$
RATE 964.8 µg/24 HOURS
AREA 4.9 cm2

Q. mg $\times 10^2$

HOURS

Figure 5

RELEASE OF BUMETANIDE FRO M THE CARBOPOL DEVICE COVERED WITH TEGADERM

SOLVATION: 25% v/v POLYSORBATE 80 AQUEOUS SOLUTION AT 30°C

European Patent Office

**EUROPEAN SEARCH REPORT**

| DOCUMENTS CONSIDERED TO BE RELEVANT | | | EP 84109290.1 |
|---|---|---|---|
| **Category** | Citation of document with indication, where appropriate, of relevant passages | Relevant to claim | **CLASSIFICATION OF THE APPLICATION (Int. Cl.4)** |
| X | PATENT ABSTRACTS OF JAPAN, unexamined applications, section C, vol. 6, no. 57, April 14, 1982 THE PATENT OFFICE JAPANESE GOVERNMENT page 21 C 98 * Kokai-no. 56-169 623 (NITTO DENKI KOGYO K.K.) * -- | 1,3,4, 11,13, 14 | A 61 K 9/26 A 61 K 9/70 |
| A | US - A - 3 803 300 (H. POSPISCHIL) * Claims 1,3,4; column 2, line 46 - column 3, line 25 * -- | 1,3,4, 6,7 | |
| A | PATENT ABSTRACTS OF JAPAN, unexamined applications, section C, vol. 7, no. 28, February 4, 1983 THE PATENT OFFICE JAPANESE GOVERNMENT page 135 C 149 * Kokai-no. 57-185 223 (KAO SEKKEN K.K.) * -- | 1,2,3, 11,13 | **TECHNICAL FIELDS SEARCHED (Int. Cl.4)** A 61 K 9/00 |
| A | GB - A - 2 042 888 (TEIJIN LTD.) * Page 3, lines 12-25; page 4, lines 12-33; page 5, lines 14-16, 34-38; page 17, new claims 1-6,9,10; page 8, lines 8-11 * -- | 1,2,18 | |

The present search report has been drawn up for all claims

| Place of search VIENNA | Date of completion of the search 13-11-1984 | Examiner MAZZUCCO |
|---|---|---|

**European Patent Office**

**EUROPEAN SEARCH REPORT**

| | DOCUMENTS CONSIDERED TO BE RELEVANT | | EP 84109290.1 |
|---|---|---|---|

| Category | Citation of document with indication, where appropriate, of relevant passages | Relevant to claim | CLASSIFICATION OF THE APPLICATION (Int. Cl.4) |
|---|---|---|---|
| A | SEIFEN-ÖLE-FETTE-WACHSE, vol. 97, no. 12, 1971, Verlag für chemische Industrie, Augsburg <br><br> M. ZIOLKOWSKY "Salbengrundlagen, die auf Schleimhäuten haften" page 426 <br><br>    * Totality * | 1,3 | |
| | GB - A - 1 465 190 (MINNESOTA MINING AND MANUFACTURING COMP.) | | |
| A | * Claims 1,3; page 4, lines 42-90; page 6, lines 31-39 * | 1-7 | |
| X | * Claims 1,3; exmaple 18 * | 8,9 | |
| A | DE - A - 2 207 294 (WARNER-LAMBERT CO.) <br><br> * Claim 1; pages 5-6 * | 1,3-5 | TECHNICAL FIELDS SEARCHED (Int. Cl.4) |
| A | GB - A - 2 023 000 (KOWA COMP. LTD.) <br><br> * Claims 1,2,4,8,9; examples 1-4 * | 1-7 | |
| D,A | EP - A2 - 0 013 606 (KEY PHARMA-CEUTICALS, INC.) <br><br> * Abstract; claim 1; page 2, line 8 - page 3, line 11; page 5, line 6 - page 6, line 17; example IV * | 1-4, 11,13, 14 | |

The present search report has been drawn up for all claims

| Place of search | Date of completion of the search | Examiner |
|---|---|---|
| VIENNA | 13-11-1984 | MAZZUCCO |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82